# EUROPÄISCHE PATENTSCHRIFT

(11) **EP 2 560 557 B1**
(45) Veröffentlichungstag und Bekanntmachung des Hinweises auf die Patenterteilung: **01.10.2014**
(21) Anmeldenummer: 11722288.5
(22) Anmeldetag: 18.03.2011
(51) Int. Cl.: A61B 17/02

(54) **ZUGVORRICHTUNG EINER CHIRURGISCHEN RETRAKTIONSVORRICHTUNG**
TENSION DEVICE OF A SURGICAL RETRACTION DEVICE
DISPOSITIF DE TRACTION D'UN DISPOSITIF DE RÉTRACTION CHIRURGICAL

(30) Priorität: 19.04.2010 DE 202010005854 U
(43) Veröffentlichungstag der Anmeldung: 27.02.2013
(73) Patentinhaber: Condor GmbH Medicaltechnik, 33154 Salzkotten (DE)
(72) Erfinder: SCHULTE, Hermann-Josef, 33154 Salzkotten (DE)
(74) Vertreter: Rolf, Gudrun
(86) Internationale Anmeldenummer: PCT/DE2011/000283
(87) Internationale Veröffentlichungsnummer: WO 2011/131162

(56) Entgegenhaltungen:
- US-A- 3 965 890
- US-A1- 2008 009 884

## Beschreibung

Die Erfindung betrifft eine Zugvorrichtung einer chirurgischen Retraktionsvorrichtung gemäß dem Oberbegriff des Hauptanspruchs.

Solche Retraktionsvorrichtungen werden im Wesentlichen für die Thorax-, Lungen- oder Herzchirurgie benötigt, um den Operationsbereich freizulegen. Eine bekannte Zugvorrichtung, US 3 965 890, weist ein Zugstück mit einem daran angeordneten Wundspreizhaken sowie ein relativ dazu ortsfestes Halteteil auf und eine dazwischen vorgesehene lineare Ratsche mit einer lösbaren Rastfunktion entgegen der Richtung der Zugkraft. Diese Zugkraft wird durch eine manuelle Betätigung, nämlich durch ein Ziehen des Zugstücks durch die Ratsche hindurch erzeugt, welche das Zugstück mechanisch gegen eine entgegengesetzte Bewegung verrastet.

Nachteilig an einer solchen bekannten Zugvorrichtung ist insbesondere, dass zu ihrer Bedienung bei der Aufspreizung des Operationsbereichs zum Teil ein erheblicher Kraftaufwand betrieben werden muss, da keine Kraftverstärkung vorhanden ist und die erforderliche Kraft jedoch nicht von jedem Operateur aufgebracht werden kann, zumal auch die zum Teil ungünstigen Platzverhältnisse einen optimalen Zugriff auf die Zugvorrichtung der Retraktionsvorrichtung verhindern.

Aufgabe der Erfindung ist es, eine Zugvorrichtung einer chirurgischen Retraktionsvorrichtung zur Verfügung zu stellen, welche sich mit wenig Kraftaufwand bequem bedienen lässt und die optimal zugänglich ist sowie nur ein geringes Bauvolumen beansprucht.

Die Lösung dieser Aufgabe ergibt sich in Verbindung mit den Oberbegriffsmerkmalen erfindungsgemäß dadurch, dass die Ratsche als lineare Vorschubmechanik ausgebildet ist und die Vorschubmechanik mit einem Betätigungshebel und einer Entriegel ausgestattet ist. Hierdurch wird es auf besonders einfache Art und Weise ermöglicht, über einen mechanischen Betätigungshebel die Strecke zwischen einem Wundspreizhaken und dessen ortsfesten Anordnung an einem Wundspreizrahmen oder einer OP-Tischhalterung zu verringern, wozu verschiedene Vorschubmechaniken verwandt werden können. Der Betätigungshebel verringert zum einen die erforderliche Kraft zum Aufspreizen des Operationsgebietes und verlagert zudem den Kraftangriffspunkt in einen ergonomisch vorteilhafteren Bereich oberhalb des OP-Tisches.

Gemäß der Erfindung ist an dem der Haltestange zugewandten Ende des Zugstücks eine Aufnahme für die Haltestange vorgesehen, wobei die Aufnahme auch gleichzeitig als Gehäuse für die Vorschubmechanik ausgeführt, welche eine Schwenkachse für den Betätigungshebel aufweist, an dem mindestens ein erstes Funktionsteil angeordnet ist, welches eine aufhebbare kraft-, form- und/ oder reibschlüssige Verbindung mit dem Zugstück erzeugt und mit dem das Zugstück einen geringen Weg in Richtung der Haltestange bewegbar ist. Im Betätigungshebel ist des Weiteren ein zweites Funktionsteil vorgesehen, welches ebenfalls eine aufhebbare kraft-, form- und/ oder reibschlüssige Verbindung mit dem Zugstück aufweist und dieses beim Zurückschwenken des Betätigungshebels gegen ein Zurückstellend es Zugstückes verriegelt.

Durch eine mehrfache Betätigung des Betätigungshebels lässt sich demnach ein Wundspreizhaken oder andere Hilfsmittel ohne großen Kraftaufwand bewegen, insbesondere ohne dass dazu eine identisch große Zugkraft manuell in Zugrichtung aufgebracht werden müsste. Das für die lineare Vorschubmechanik zusätzlich benötigte Bauvolumen ist dagegen annähernd vernachlässigbar.

Entsprechend der Erfindung besteht das erste Funktionsteil aus einem Rasthebel mit einer Rastnase, welcher etwa parallel zur Haltestange federvorgespannt im Betätigungshebel verschwenkbar gelagert ist. Die Haltestange ist über ihre Länge mit einer Rastenbahn ausgestattet und die Rastnase des Rasthebels greift in diese Rastenbahn ein. Das zweite Funktionsteil besitzt ebenfalls eine Rastnase, wobei die erste Rastnase eine Bewegung des Zugstücks in Richtung der Haltestange erlaubt und die zweite Rastnase eine Bewegung des Zugstücks entgegen der Zugkraft verriegelt.

Eine solch einfach wirkende lineare Ratsche ist erfindungsgemäß dadurch erheblich verbessert, dass die Haltestange über ihre Länge beidseitig mit sich gegenüberliegend angeordneten Rastenbahnen ausgestattet ist und der Betätigungshebel die Haltestange umgreift und das zweite Funktionsteil der gegenüberliegenden Rastenbahn zugeordnet ist, wobei dabei das erste und zweite Funktionsteil beide identisch als Rasthebel mit Rastnasen ausgebildet sind, so dass sie ihre Funktion in Abhängigkeit vom Schwenkwinkel des Betätigungshebels wechseln können, wodurch es vorteilhaft möglich wird, dass bei jedem Hub des Betätigungshebels das Zugstück um einen kleinen Betrag in Richtung der Haltestange bewegt wird. Hierdurch wird eine kontinuierlichere und gleichmäßigere Aufspreizung eines Operationsbereichs erreicht, wobei der Kraftaufwand in beide Hubbewegung des Betätigungshebels identisch ist, was die Handhabung der Vorrichtung weiter verbessert.

Die beiden Rasthebel sind vorteilhaft auf ihren beiden den Rastnasen gegenüberliegenden Enden mit Hebelenden ausgestattet, über die die Rastnasen durch eine gleichzeitige Verschwenkung um ihre Lagerachsen im Betätigungshebel gegen eine elastische Rückstellkraft aus ihrer Verrastung bewegbar sind, so dass eine Entriegelung des Zugstückes und damit eine Entlastung des Wundspreizhakens schnell und einfach möglich ist.

Die Schwenkachse des Betätigungshebels wird bevorzugt von zwei sich beidseitig der Haltestange durch den Kopf des Betätigungshebels bis in die Haltestange erstreckende Führungsbolzen gebildet, wobei die Haltestange beidseitig über ihre Länge mit Führungsnuten versehen ist, in die diese Führungsbolzen eingreifen. Hierdurch wird zum einen eine sicherere Verdrehsicherung der Haltestange in der Aufnahme des Zugstückes erreicht sowie eine Bewegungsbegrenzung des Zugstücks gewährleistet, wobei die Führungsbolzen einen weiteren Vorteil der erfinderischen Zugvorrichtung dadurch erzielen, dass sie in der Aufnahme nur verrastet und daraus herausziehbar ausgebildet sind, so dass die gesamte Zugvorrichtung zu Reinigungszwecken auf einfachste Art und Weise zerlegbar ist.

Der Wundspreizhaken ist vorteilhaft mit seinem zylindrischen Haltearm in einer weiteren Aufnahme des Zugstücks verrastbar, in dem ein federgelagerter Entriegelungsstift zur Einführung des Haltearmes gedrückt und zur Verriegelung losgelassen werden muss, wobei er mit seiner Außenkontur in eine radiale Ausnehmung im Haltearm eingreift und diesen verriegelt.

Nachfolgend ist ein Ausführungsbeispiel der Erfindung anhand von Zeichnungen näher beschrieben. Es zeigen:

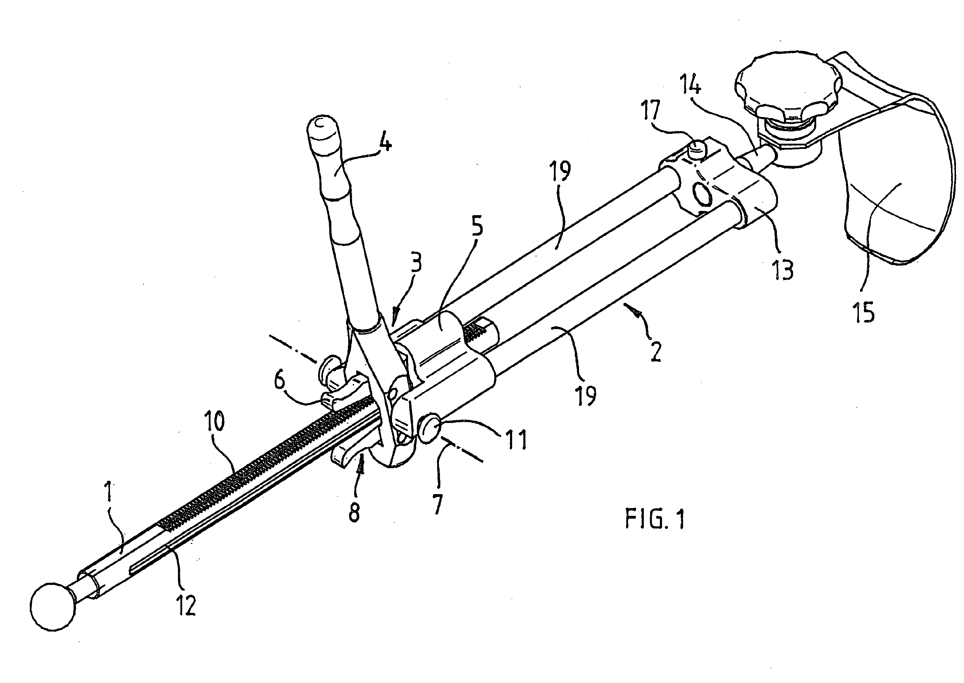

- Fig. 1: eine räumliche Ansicht der Zugvorrichtung mit eingesetztem Wundhaken,

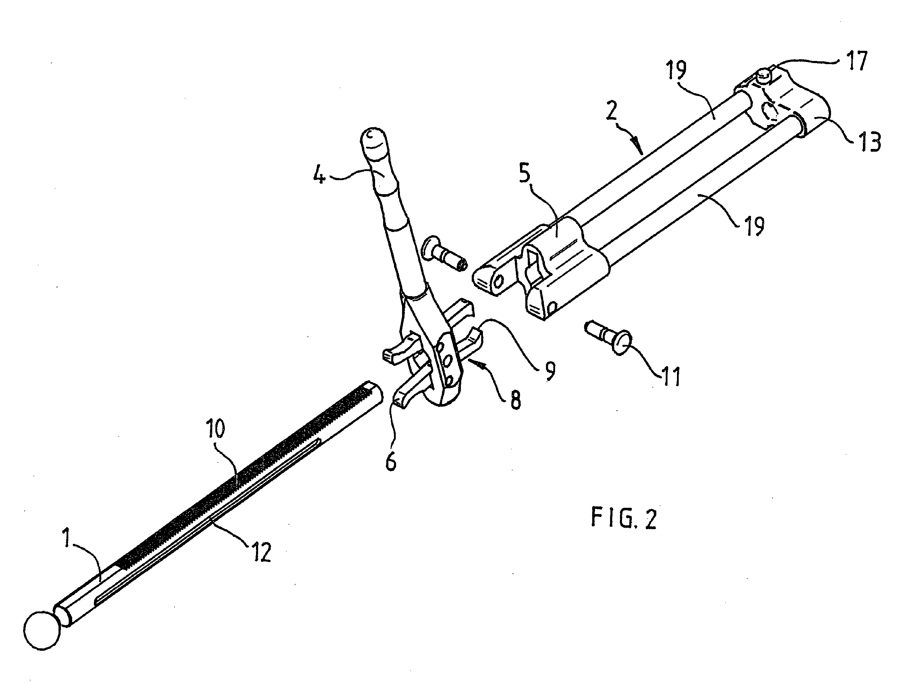

- Fig. 2: eine Explosionsdarstellung der Zugvorrichtung gem. Fig. 1,

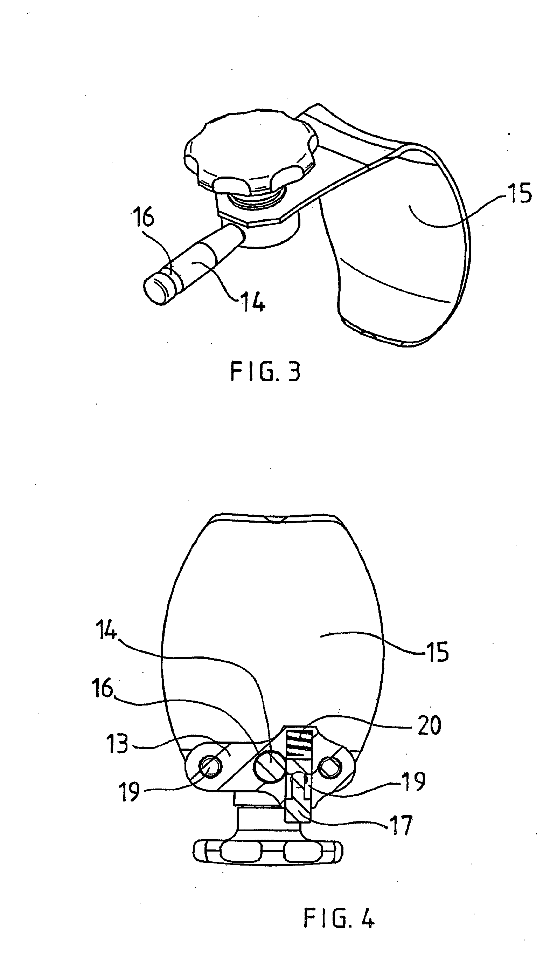

- Fig. 3: eine räumliche Ansicht eines Wundspreizhakens, und
- Fig. 4: einen Schnitt durch die Aufnahme des Wundspreizhakens gem. Fig. 3.

Die Zugvorrichtung einer chirurgischen Retraktionsvorrichtung besteht im Wesentlichen aus einer ortsfest mittelbar oder unmittelbar an einem Wundspreizrahmen oder einem OP-Tisch-Ständer angeordneten Haltestange 1 und einem dazu beweglichen Zugstück 2, welches an seinem der Haltestange 1 abgewandten Ende mit einer Aufnahme 13 für einen Wundspreizhaken 15 ausgestattet ist, der mit einem Haltearm 14 in einer entsprechenden Bohrung der Aufnahme 13 angeordnet und dort von einem Ver- und Entriegelungsstift 17 lösbar gehalten ist, wobei der Haltearm 14 des Wundspreizhakens 15 eine radiale Ausnehmung 16 aufweist, in die die Körperkontur des Ver- und Entriegelungsstiftes 17 in einer Verriegelungsstellung eingreift bzw. in einer gegen die Federkraft einer Feder 20 gedrückten Entriegelungsposition über eigene radiale Ausnehmungen 18 den Haltearm 14 zur Montage oder Demontage freigibt.

Die Haltestange 1 weist zwei sich gegenüberliegend angeordnete Rastbahnen 10 auf, die sich über ihren Großteil ihrer Länge erstrecken sowie rechtwinklig dazu zwei sich gegenüberliegend angeordnete Führungsnuten 12, in die sich Führungsbolzen 11 hineinerstrecken und so zum einen eine Verdrehsicherung der Haltestange 1 und zum anderen eine Bewegungsbegrenzung der Haltestange 1 bilden. Die Führungsbolzen 11 selbst weisen radiale Ausnehmungen auf, mittels derer sie über radial auf die führungsbolzen 11 einwirkende Federkugeln lösbar in der Aufnahme 3 der Zugvorrichtung gelagert sind.

Die Führungsbolzen 11 durchgreifen dabei den Kopfteil des Betätigungshebels 4 und bilden so dessen Schwenkachse 7. Etwa parallel zu den Ratenbahnen 10 sind Rasthebel 8 verschwenkbar im Spannhebel 4 gelagert, wo sie federunterstützt mit ihren Rastnasen 9 in Rasten der Rastbahn 10 eingedrückt gehalten werden. An ihren den Rastnasen 9 entgegen gesetzten Enden sind die Rasthebel 8 als Hebelenden 6 ausgeführt, die durch eine Schwenkbewegung in Richtung auf die Rastbahnen die Rastnasen 9 aus ihrer Verrastung heben und so ein Zurückführen des Zugstücks 2 in eine Ausgangslage ermöglichen.

Diese Vorschubmechanik funktioniert als lineare, doppelseitig wirkende Ratsche, die bei einer Schwenkbewegung des Betätigungshebels 4 in Richtung des Wundspreizhakens mit ihrem dem Betätigungshebel 4 zugewandten Rasthebel 8 das Zugstück 2 um einige Rasten weiter in Richtung der Haltestange 1 bewegt, wobei der gegenüberliegende Rasthebel 8 diese Anzahl von Rasten seiner Rastbahn 10 überspringt und bei einer Gegenbewegung des Betätigungshebels 4 in Rasten der Rastenbahn 10 einrastet und seinerseits das Zugstück 2 um einige Rasten näher an die Haltestange 1 bewegt, wobei der auf der gegenüberliegenden Seite angeordnete Rasthebel 8 nun seinerseits einige Rasten seiner Rasenbahn 10 überspringt.

Zur Entriegelung der Mechanik werden die beiden Hebelenden 6 der Rasthebel 8 gemeinsam in Richtung der Rastbahnen 10 gedrückt, wobei beide Rastnasen 9 der Rasthebel 8 außer Eingriff geraten, so dass das Zugstück 2 widerstandslos ausgezogen werden kann. Das Zugstück 2 selber weist neben der Aufnahme 13 für den Wundspreizhaken 15 und die Aufnahme 3 für die Ratschenmechanik zwei parallel beabstandete Stäbe 19 auf, zwischen die die Haltestange 1 bewegbar ist, so dass seine symmetrische Kraftverteilung in der gesamten Zugvorrichtung aufrechterhalten ist. Zur Voreinstellung der Zugvorrichtung lässt sich das Zugstück 2 samt Wundspreizhaken 15 gegen die überspringenden Rastnasen 9 der Rasthebel 8 bewegen und durch den Betätigungshebel 4 eine Feinjustierung der Retraktionsvorrichtung erreichen.

## Patentansprüche

1. Zugvorrichtung einer chirurgischen Retraktionsvorrichtung mit einem Zugstück (2) zum Aufbringen einer Zugkraft auf ein daran anzuordnendes Werkzeug wie einen Wundspreizhaken (15) und mit einer relativ zum Zugstück (2) ortsfesten Haltestange (1) sowie mit einer dazwischen vorgesehenen lineare Ratsche mit einer lösbaren Rastfunktion entgegen der Richtung der Zugkraft, **dadurch gekennzeichnet, dass** die Ratsche als lineare Vorschubmechanik (3) ausgebildet ist und die Vorschubmechanik (3) einen Betätigungshebel (4) und eine Entriegelung aufweist und an dem der Haltestange (1) zugewandten Ende des Zugstücks (2) eine Aufnahme (5) für die Haltestange (1) und als Gehäuse für die Vorschubmechanik (3) vorgesehen ist, welches eine Schwenkachse (7) für den Betätigungshebel (4) aufweist und an dem mindestens ein erstes Funktionsteil angeordnet ist, welches eine aufhebbare kraft-, form- und/ oder reibschlüssige Verbindung mit dem Zugstück (2) erzeugt und mit dem das Zugstück (2) in Richtung der Haltestange (1) bewegbar ist, sowie ein zweites Funktionsteil, weiches ebenfalls eine aufhebbare kraft-, form- und/ oder reibschlüssige Verbindung mit dem Zugstück (2) aufweist und dieses beim Zurückschwenken des Betätigungshebel (4) gegen eine Rückstellung des Zugstückes (2) verriegelt hält und das erste Funktionsteil aus einem Rasthebel (8) mit einer Rastnase (9) besteht, welcher etwa parallel zur Haltestange (1) federvorgespannt verschwenkbar im Betätigungshebel (4) gelagert ist und die Haltestange (1) über ihre Länge mit einer Rastenbahn (10) ausgestattet ist und die Rastnase (9) des Rasthebels (8) in die Rastenbahn (10) eingreift und das zweite Funktionsteil ebenfalls eine Rastnase (9) aufweist und die Haltestange (1) über ihre Länge beidseitig mit sich gegenüberliegend angeordneten Rastbahnen (10) ausgestattet ist, der Betätigungshebel (4) die Haltestange (1) umgreift und das zweite Funktionsteil auf der gegenüberliegenden Rastenbahn (10) eingreift und das beide Funktionsteil identisch als Rasthebel (8) mit Rastnasen (9) ausgebildet sind und die Rasthebel (8) ihre Funktion in Abhängigkeit von der Schwenkrichtung des Betätigungshebel (4) wechseln.

2. Zugvorrichtung nach Anspruch 1, **dadurch gekennzeichnet, dass** jeder Rasthebel (8) auf seinem der Rastnase (9) gegenüber liegenden Ende ein Hebelende (6) aufweist, über das die Rastnase (9) durch eine Verschwenkung um eine Schwenkachse des Rasthebels (8) im Betätigungshebel (4) gegen eine elastische Rückstellkraft aus ihrer Rastung bewegbar ist.

## Claims

1. Tensioning device of a surgical retractor, with a tensioning section (2) for applying a tensile force to a tool that is to be arranged thereon, for example a retractor hook (15), and with a holding bar (1) fixed in position relative to the tensioning section (2), and also with a linear ratchet, which is provided between the holding bar (1) and the tensioning section (2) and has a releasable locking function counter to the direction of the tensile force, **characterized in that** the ratchet is designed as a linear feed mechanism (3) and the feed mechanism (3) has an actuating lever (4) and an unlocking means, on the end of the tensioning section (2) directed toward the holding bar (1), a receiver (5) is provided for the holding bar (1) and as a housing for the feed mechanism (3), which housing has a pivot axis (7) for the actuating lever (4) and on which at least one first function part is arranged which generates a force-fit, form-fit and/or friction- fit connection, that can be cancelled, to the tensioning section (2) and with which the tensioning section (2) can be moved in the direction of the holding bar (1), and a second function part, which likewise has a force-fit, form-fit and/or friction-fit connection, that can be cancelled, to the tensioning section (2) and keeps the latter locked, when the actuating lever (4) is pivoted back, counter to a restoring of the tensioning section (2), and the first function part is composed of a locking lever (8), which has a locking lug (9) and is pretensioned by a spring approximately parallel to the holding bar (1) and is mounted pivotably in the actuating lever (4), and the holding bar (1) is provided along the length thereof with a latching track (10), and the locking lug (9) of the locking lever (8) engages in the latching track (10), and the second function part likewise has a locking lug (9) and the holding bar (1) is provided on both sides along its length with latching tracks (10) arranged opposite each other, the actuating lever (4) engages around the holding bar (1), and the second function part engages on the opposite latching track (10), and **in that** both function parts are designed identically as locking levers (8) with locking lugs (9), and the locking levers (8) alternate in their function depending on the direction of pivoting of the actuating lever (4).

2. Tensioning device according to Claim 1, **characterized in that** each locking lever (8), at its end opposite the locking lug (9), has a lever end (6) via which the locking lug (9), by a pivoting movement about a pivot axis of the locking lever (8) in the actuating lever (4), is movable from its locking engagement counter to an elastic restoring force.

## Revendications

1. Dispositif de traction d'un dispositif de rétraction chirurgical comprenant une pièce de traction (2) pour appliquer une force de traction à un outil devant être disposé contre celle-ci, tel qu'un crochet écarteur (15), et comprenant une tige de retenue (1) fixe par rapport à la pièce de traction (2) ainsi qu'un cliquet linéaire prévu entre celles-ci, ayant une fonction d'encliquetage détachable à l'encontre de la direction de la force de traction, **caractérisé en ce que** le cliquet est réalisé sous forme de mécanisme d'avance linéaire (3) et le mécanisme d'avance (3) présente un levier d'actionnement (4) et un déverrouillage et, au niveau de l'extrémité de la pièce de traction (2) tournée vers la tige de retenue (1), un logement (5) est prévu pour la tige de retenue (1) et servant de boîtier pour le mécanisme d'avance (3), lequel présente un axe de pivotement (7) pour le levier d'actionnement (4) et sur lequel est disposée au moins une première partie fonctionnelle, laquelle produit une connexion pouvant être supprimée par engagement par force, par correspondance de forme et/ou par friction avec la pièce de traction (2) et avec laquelle la pièce de traction (2) peut être déplacée dans la direction de la tige de retenue (1), ainsi qu'une deuxième partie fonctionnelle, laquelle présente également une connexion pouvant être supprimée par engagement par force, par correspondance de forme et/ou par friction avec la pièce de traction (2) et retient celle-ci de manière verrouillée lors du retour par pivotement du levier d'actionnement (4) à l'encontre d'un rappel de la pièce de traction (2), et la première partie fonctionnelle se compose d'un levier d'encliquetage (8) muni d'un ergot d'encliquetage (9) qui est monté approximativement parallèlement à la tige de retenue (1) de manière précontrainte par ressort et de manière à pouvoir pivoter dans le levier d'actionnement (4) et la tige de retenue (1) est munie le long de sa longueur d'une piste d'encliquetage (10) et l'ergot d'encliquetage (9) du levier d'encliquetage (8) s'engage dans la piste d'encliquetage (10) et la deuxième partie fonctionnelle présente également un ergot d'encliquetage (9) et la tige de retenue (1) est munie le long de sa longueur des deux côtés avec des pistes d'encliquetage (10) disposées à l'opposé l'une de l'autre, le levier d'actionnement (4) vient en prise autour de la tige de retenue (1) et la deuxième partie fonctionnelle vient en prise sur la piste d'encliquetage opposée (10) et les deux parties fonctionnelles sont réalisées de manière identique en tant que levier d'encliquetage (8) avec des ergots d'encliquetage (9) et les leviers d'encliquetage (8) changent de fonction en fonction du sens de pivotement du levier d'actionnement (4).

2. Dispositif de traction selon la revendication 1, **caractérisé en ce que** le levier d'encliquetage (8) présente à chaque fois sur son extrémité opposée à l'ergot d'encliquetage (9) une extrémité de levier (6) par le biais de laquelle l'ergot d'encliquetage (9) peut être déplacé hors de sa position encliquetée par pivotement autour d'un axe de pivotement du levier d'encliquetage (8) dans le levier d'actionnement (4) à l'encontre d'une force de rappel élastique.
